# EUROPEAN PATENT APPLICATION

(11) **EP 4 467 106 A1**
(43) Date of publication of application: **27.11.2024**
(21) Application number: 23020241.8
(22) Date of filing: 23.05.2023
(51) Int. Cl.: A61F 2/38

(54) **TOTAL KNEE ENDOPROSTHESIS**

(71) Applicant: Medizinische Universität Graz, 8010 Graz (AT)
(72) Inventor: Sadoghi, Patrick, 8010 Graz (AT)
(74) Representative: SONN Patentanwälte GmbH & Co KG

(57) **Abstract**

In a total knee endoprosthesis (1) comprising a tibial component (2) and a femoral component (3) and a sliding plate (4) arranged between the tibial component (2) and the femoral component (3), the femoral component (3) having medial (5) and lateral articulation surfaces (6) of a femoral shield (9) resting on medial (5) and lateral articulation surfaces (6) of the sliding plate (4), wherein the tibial component (2) and the femoral component (3) are mechanically coupled for pivoting, the mechanical coupling is effected in the area of the medial articulation surfaces (5) via a first coupling element (7) and in the area of the lateral articulation surfaces (6) via a second coupling element (8).

## Description

The present invention relates to a total knee endoprosthesis comprising a tibial component and a femoral component and a sliding plate arranged between the tibial component and the femoral component, the femoral component having medial and lateral articulation surfaces of a femoral shield resting on medial and lateral articulation surfaces of the sliding plate, wherein the tibial component and the femoral component are mechanically coupled for pivoting.

Total knee endoprostheses of this kind are known in the prior art as fully coupled replacement- or fully coupled revision total knee endoprostheses. After multiple revisions of a total knee endoprosthesis or after injuries to the cruciate and/or collateral ligaments, reconstruction of a functional joint is only possible with a fully coupled replacement total knee endoprosthesis. The mechanical coupling of the femoral with the tibial components of these fully coupled total knee endoprostheses is necessary because without the existing ligament system, the knee would be unstable in a varus or valgus or in an anterior or posterior direction. In the prior art, the coupling mechanism of such fully coupled total knee endoprostheses is essentially a hinge, which is mounted in the tibial component in a way that it can be rotated by a certain degree, so that in addition to bending (flexion and extension) of the leg treated in this way, there is also a certain degree of external and internal rotation about the vertical leg axis available.

A fully coupled total knee endoprosthesis of the above-mentioned type can be seen, for example, in DE 4102509 A1.

With the known types of coupling, however, flexion and extension take place without the physiological roll-slide mechanism, which leads to early loosening of the prosthesis from the bone interface. This means that with fully constrained or fully coupled total knee endoprostheses, due to the non-physiological forces occurring, there is increased loosening of the shafts that serve to support the fully coupled total knee endoprostheses in the medullary cavity.

It is therefore the object of the present invention to improve a total knee endoprosthesis of the type mentioned at the outset in such a way that the physiological movement pattern can be maintained or restored, so that better healing, faster familiarization with the prosthesis and lower loosening rates in the long term can be achieved.

To solve this problem, a total knee endoprosthesis of the type mentioned at the outset according to the invention is characterized in that the mechanical coupling is effected in the area of the medial articulation surfaces via a first coupling element and in the area of the lateral articulation surfaces via a second coupling element.

Instead of the central hinge of conventional fully coupled total knee endoprostheses which, in contrast to the physiological anatomy, allows only for central rotation instead of a natural rolling and sliding movement of the knee joint, the mechanical coupling of the tibial and femoral components in the present invention is accomplished by two independent coupling elements. In this way, a different type of mechanical coupling can be used in the area of the medial articulation surfaces (medial compartment) than in the area of the lateral articulation surfaces (lateral compartment), so that a joint function can be realized that not only allows flexion and extension as well as an external and internal rotation of the joint, but in particular simulates the roll-slide mechanism of the knee during flexion.

According to a preferred embodiment of the present invention, the first coupling element is formed by a ball head which engages behind the femoral shield in the area of the medial articulation surface or is guided in a covered channel of the same, and the ball head is connected to the tibial component through the medial articulation surfaces of the femoral shield and the sliding plate in a tension-fast manner. This closely resembles the physiology of the knee joint in the medial compartment and already provides the ability to flex and extend the knee, as well as some internal and external rotation. At the same time, the tension-fast connection between the ball head and the tibial component prevents the knee joint from tilting into a valgus. The medial compartment is therefore physiologically reproduced with this coupling element. Under certain circumstances, the ball head can also be designed hemispherically, in which case the spherical geometry must be realized in a caudal area, i.e. in an area of the ball head facing the femur shield.

To connect the ball head to the tibial component, a preferred embodiment of the present invention provides that the femoral shield has a first slot in the region of the medial articulation surface in a direction from caudal to posterior for the passage of a connecting rod, preferably designed in one piece with the ball head, which extends from the ball head to the tibial component, wherein the first slot extends at least from a caudal portion of the femoral shield to the posterior end of the femoral shield. The first slot thus defines a wide range of flexion of the medial compartment and allows both full flexion of the knee joint and end-rotation of the knee joint for comfortable standing with the knee extended. The connecting rod can either be connected to the ball head, for example by screwing, and in this way its length can be selected depending on the dimensions of the knee to be treated, or the connecting rod can be designed in one piece with the ball head in order to ensure a connection between the ball head and the knee that is as durable as possible to ensure long-term strength.

The first slot is preferably designed to be open at the posterior end of the femoral shield, which can be used to guide the ball head during surgery to implant the inventive fully coupled total knee endoprosthesis, from the posterior end to a position behind the femoral shield already connected to the bone in order to engage the ball head behind the femoral shield.

In order to realize the physiological roll-slide mechanism in the lateral compartment, the inventive total knee endoprosthesis preferably is characterized in that the second coupling element is formed by a covered guide channel, running flush in a direction from caudal to posterior in the femoral shield and a guide piece guided in the guide channel, the guide piece being connected to the tibial component through a second slot in the lateral articulating surface of the femoral shield and through the sliding plate. In connection with the present invention, a covered channel is a channel that runs inside the femoral shield and is thus closed off to the outside in such a way that the guide piece does not move backwards, i.e. not in the direction of the side of the femoral shield facing away from the articulation surface, i.e. the guide piece cannot exit in cranial and ventral directions. The second coupling element secures the leg treated in this way against tilting into a varus and at the same time allows a combined rolling and sliding movement in the medial compartment, so that a largely physiological flexion pattern of the inventive total knee endoprosthesis can be achieved. Like the first slot, the second slot provides sufficient flexion range. It goes without saying that in this preferred embodiment the covered guide channel and the second slot run over the same length and in the same direction.

As already described in connection with the medial compartment, the guide channel and the second slot extend in a caudal to posterior direction, wherein the second slot extends at least from a caudal region of the femoral shield to the posterior end of the femoral shield, as it is in conformity with a preferred embodiment of the present invention.

According to a preferred embodiment of the present invention, the covered guide channel in the femoral shield, starting from a first depth below the lateral articulation surface in the caudal area of the femoral shield, extends to a second depth, different from the first depth, below the lateral articulation surface in the posterior area of the femoral shield, with preferably the first depth in relation to the lateral articulation surface being between 5 mm and 3 mm, preferably 3 mm deeper in the femoral shield than the second depth. In this way, the physiological lateral lift-off in flexion is restored.

According to a preferred embodiment of the present invention, the guide piece is designed as a sliding piece. Alternatively and in a likewise preferred manner, however, the guide piece can also have rollers and can, thus, be designed as a rolling guide piece in the covered guide channel. In this way, the guide piece is guided in the channel with less wear.

Preferably, the second slot and the guide channel are designed to be open at the posterior end of the femoral shield, which can be used to guide the guide piece from the rear, i.e. from the posterior end, into the guide channel during surgery to implant the inventive fully coupled total knee endoprosthesis, in order to accomplish coupling of the second coupling element.

Since with a fully coupled total knee endoprosthesis there is usually no functional ligamentous system at the knee joint, all forces must be absorbed by the total knee endoprosthesis. For this purpose, the tibial and/or the femoral component can each be connected to an anchoring shaft for anchoring the respective component in a bone. Depending on the anatomical situation, a suitable anchoring shaft can be selected for the tibial and femoral components, with these being available in different lengths and thicknesses and being screwed to the respective component, if necessary with the interposition of so-called offset pieces. In the inserted state, the aim is then to ensure that the tibial and femoral component fit as closely as possible to the shape of the correspondingly prepared condyle and, at the same time, to ensure that the anchoring shaft is firmly seated in the metaphysis or in the diaphysis of the respective bone.

Preferably, the components of the total knee endoprosthesis, with the exception of the sliding plate, are made of surgical steel and/or a chromium-cobalt-molybdenum alloy and preferably are provided with an abrasion-resistant coating, in particular a titanium nitride coating or a niobium-titanium coating.

Titanium nitride coatings and niobium-titanium coatings are valued for their hypoallergenic effects, and the correspondingly coated components of the fully coupled total knee endoprosthesis according to the invention are consequently characterized by particularly good tolerability and rapid healing-in of the implant.

The invention will now be exemplified in more detail with reference to an embodiment shown in the drawing. In the drawing, Fig. 1 shows a symbolic representation of the fully coupled total knee endoprosthesis according to the invention in a front view, Figs. 2 and 3 show corresponding views from medial and lateral sides , Fig. 4 shows an axial top view of a tibial component of the inventive total knee endoprosthesis, Fig. 5 a view of the caudal area of the femoral shield of the femoral component of the inventive total knee endoprosthesis, and Figs. 6a and 6b are illustrations of the process of coupling the components of the inventive total knee endoprosthesis.

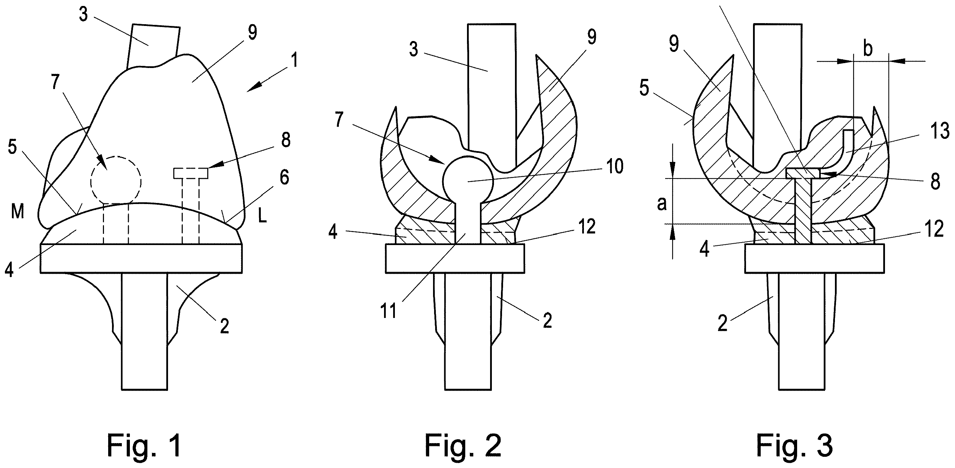

In Fig. 1, the total knee endoprosthesis according to the invention is denoted by the reference numeral 1 and consists essentially of a tibial component 2, a femoral component 3 and a sliding plate 4 arranged between the components 2 and 3. The components 2 and 3 and the sliding plate 4 have medial articulation surfaces 5 and lateral articulation surfaces 6, whereby a medial compartment M and a lateral compartment L of the inventive total knee endoprosthesis 1 are defined. It can be seen in Fig. 1 that the mechanical coupling in the area of the medial articulation surfaces 5 is effected via a first coupling element 7 and in the area of the lateral articulation surfaces 6 via a second coupling element 8.

In the representation according to Figure 2, the first coupling element 7, i.e. the medial coupling element 7, is shown as a ball head 10 which engages behind the femoral shield 9 of the femoral component 3, with a connecting rod 11 which is designed in one piece with the ball head 10 being provided to connect the ball head 10 to tibial component 2 in a tension-fast manner. A support plate of the tibial component 2 for the sliding plate 4 is denoted by reference number 12.

In contrast to this, the second coupling element 8, i.e. the lateral coupling element 8, is formed by a covered guide channel 13 in the femoral shield 9 and a guide piece 14 guided in the guide channel 13, as can be seen in Fig. 3. Here, the covered guide channel 13 in the femoral shield 9 extends from a first depth a below the lateral articulation surface 5 in the caudal area of the femoral shield 9 to a second depth b that differs from the first depth a, wherein the first depth a in relation to the lateral articulation surface 5 being approximately 3 mm deeper in the femoral shield 9 than the second depth b. This restores the physiological lateral lift-off in flexion.

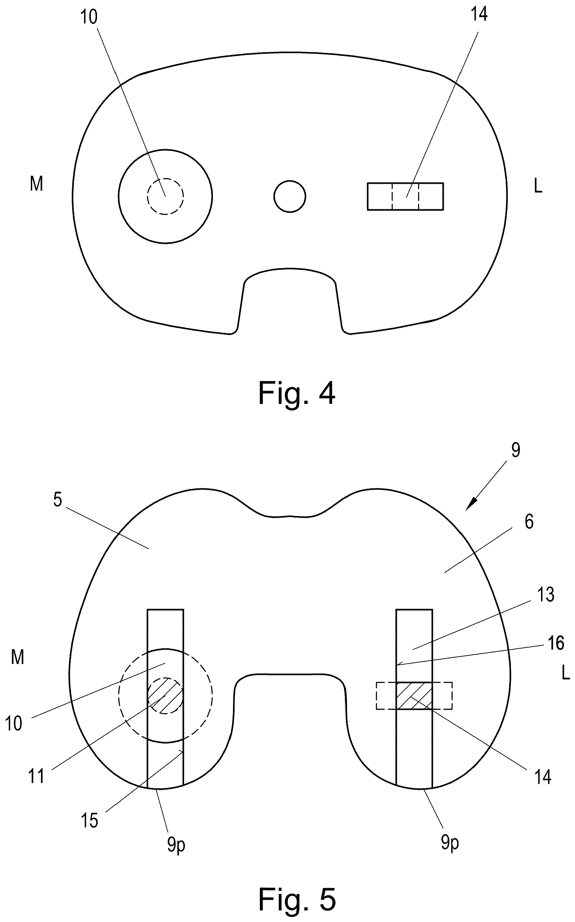

In the depiction according to Fig. 4 it can be seen that the guide piece 14 is formed by a type of beam which can engage in the guide channel 13 and that the ball head 10 has a circular geometry in a plan view. Under certain circumstances, the ball head 10 can also be designed in the form of a hemisphere.

In Fig. 5 it can be seen that the femoral shield 9 has a first slot 15 in the region of the medial articulation surface 5 in a direction from caudal to posterior for the passage of the connecting rod 11, which extends from the ball head 10 to the tibial component. The first slot 15 extends from a caudal area of the femoral shield 9 to the posterior end 9p of the femoral shield 9. Also, a slot 16 is arranged in the area of the lateral articulation surface 6 of the femoral shield 9, which allows the guide piece 14 to pass through. The guide channel 13 and the second slot 16, just as the first slot 15, extend in a direction from caudal to posterior, with the second slot 16 also extending from a caudal area of the femoral shield 9 to the posterior end 9p of the femoral shield 9 .

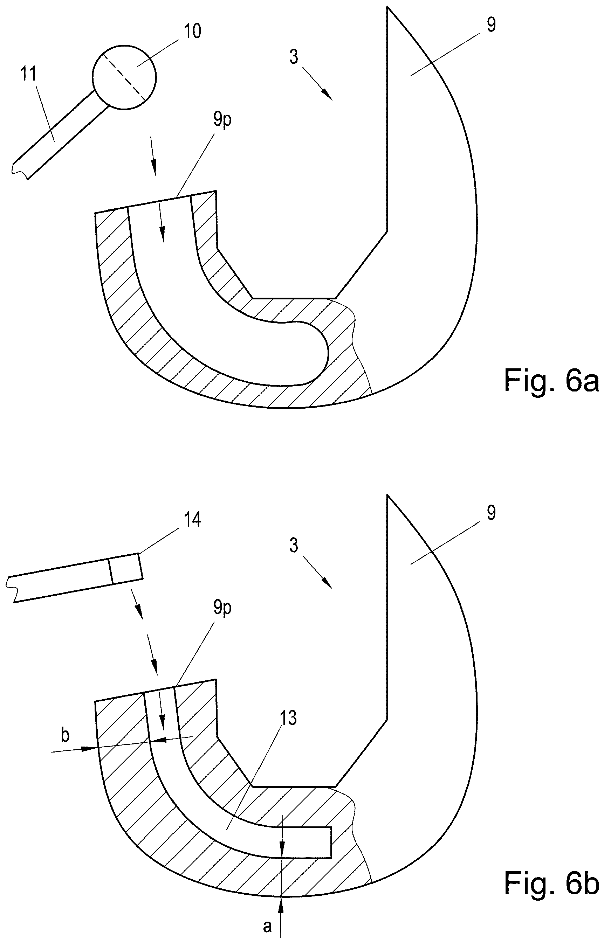

It can be seen in Figs. 6a and 6b that both the ball head 10 and the guide piece 14 can be brought into engagement with the femoral component 3 from the posterior side, i.e. from the posterior end 9p of the femoral shield 9.

## Claims

1. Total knee endoprosthesis (1) comprising a tibial component (2) and a femoral component (3) and a sliding plate (4) arranged between the tibial component (2) and the femoral component (3), the femoral component (3) having medial (5) and lateral articulation surfaces (6) of a femoral shield (9) resting on medial (5) and lateral articulation surfaces (6) of the sliding plate (4), wherein the tibial component (2) and the femoral component (3) are mechanically coupled for pivoting, **characterized in that** the mechanical coupling is effected in the area of the medial articulation surfaces (5) via a first coupling element (7) and in the area of the lateral articulation surfaces (6) via a second coupling element (8).

2. Total knee endoprosthesis according to claim 1, **characterized in that** the first coupling element (7) is formed by a ball head (10) which engages behind the femoral shield (9) in the area of the medial articulation surface (5) or is guided in a covered channel of the same, and the ball head (10) is connected to the tibial component (2) through the medial articulation surfaces (5) of the femoral shield (9) and the sliding plate (4) in a tension-fast manner.

3. Total knee endoprosthesis according to claim 2, **characterized in that** the femoral shield (9) has a first slot (15) in the region of the medial articulation surface (5) in a direction from caudal to posterior for the passage of a connecting rod (11), preferably designed in one piece with the ball head (10) , which extends from the ball head (10) to the tibial component (2) wherein the first slot (15) extends at least from a caudal portion of the femoral shield (9) to the posterior end (9p) of the femoral shield (9).

4. Total knee endoprosthesis according to claim 3, **characterized in that** the first slot (15) is designed to be open at the posterior end (9p) of the femoral shield (9).

5. Total knee endoprosthesis according to any one of claims 1 to 4, **characterized in that** the second coupling element (8) is formed by a covered guide channel (13), running flush in a direction from caudal to posterior in the femoral shield (9) and a guide piece (14) guided in the guide channel (13), the guide piece (14) being connected to the tibial component (2) through a second slot (16) in the lateral articulating surface (6) of the femoral shield (9) and through the sliding plate (4) .

6. Total knee endoprosthesis according to claim 5, **characterized in that** the guide channel (13) and the second slot (16) extend in a caudal to posterior direction, wherein the second slot (16) extends at least from a caudal region of the femoral shield (9) to the posterior end (9p) of the femoral shield (9).

7. Total knee endoprosthesis according to claim 5 or 6, **characterized in that** the covered guide channel (13) in the femoral shield (9), starting from a first depth (a) below the lateral articulation surface (6) in the caudal area of the femoral shield (9), extends to a second depth (b), different from the first depth (a), below the lateral articulation surface (6) in the posterior area of the femoral shield (9).

8. Total knee endoprosthesis according to claim 7, **characterized in that** the first depth (a) in relation to the lateral articulation surface (6) is between 5 mm and 3 mm, preferably 3 mm deeper in the femoral shield (9) than the second depth (b).

9. Total knee endoprosthesis according to one of claims 5 to 8, **characterized in that** the guide piece (14) is designed as a sliding piece.

10. Total knee endoprosthesis according to one of claims 5 to 8, **characterized in that** the guide piece (14) has rollers.

11. Total knee endoprosthesis according to one of claims 5 to 10, **characterized in that** the second slot (16) and the guide channel (13) are designed to be open at the posterior end (9p) of the femoral shield (9).

12. Total knee endoprosthesis according to one of claims 1 to 11, **characterized in that** the tibial (2) and/or the femoral component (3) can each be connected to an anchoring shaft for anchoring the respective component (2, 3) in a bone.

13. Total knee endoprosthesis, **characterized in that** the components of the total knee endoprosthesis, with the exception of the sliding plate (4), are made of surgical steel and/or a chromium-cobalt-molybdenum alloy and preferably are provided with an abrasion-resistant coating, in particular a titanium nitride coating or a niobium-titanium coating.
